# EUROPEAN PATENT SPECIFICATION

(11) **EP 2 385 986 B1**
(45) Date of publication and mention of the grant of the patent: **08.06.2016**
(21) Application number: 09804198.1
(22) Date of filing: 31.12.2009
(51) Int. Cl.: C12N 15/82, A01H 5/00

(54) **TRANSPLASTOMIC PLANTS FREE OF THE SELECTABLE MARKER**
TRANSPLASTOME PFLANZEN OHNE DEN SELEKTIONIERBAREN MARKER
Plantes transplastomiques dépourvues du marqueur sélectionnable

(30) Priority: 07.01.2009 EP 09356001; 10.07.2009 US 224508 P
(43) Date of publication of application: 16.11.2011
(73) Proprietor: Bayer Intellectual Property GmbH, 40789 Monheim (DE)
(72) Inventor: LESTRADE, Christelle, F-82600 Verdun sur Garonne (FR); PELISSIER, Bernard, F-69370 Saint-Didier-au-Mont-d'Or (FR); ROLAND, Anne, F-69270 Fontaines-sur-Rhône (FR); DUBALD, Manuel, Raleigh, NC 27612 (US)
(74) Representative: Guitton, Carole
(86) International application number: PCT/EP2009/068043
(87) International publication number: WO 2010/079117

(56) References cited:
- EP-A1- 2 006 387
- WO-A-01/81600
- WO-A-2004/053133
- WO-A-2006/072607
- DUFOURMANTEL NATHALIE ET AL: "Generation and characterization of soybean and marker-free tobacco plastid transformants over-expressing a bacterial 4-hydroxyphenylpyruvate dioxygenase which provides strong herbicide tolerance." PLANT BIOTECHNOLOGY JOURNAL JAN 2007, vol. 5, no. 1, January 2007 (2007-01), pages 118-133, XP002531775 ISSN: 1467-7652 cited in the application
- LUTZ ET AL: "Construction of marker-free transplastomic plants" CURRENT OPINION IN BIOTECHNOLOGY, LONDON, GB, vol. 18, no. 2, 17 April 2007 (2007-04-17), pages 107-114, XP022032088 ISSN: 0958-1669
- LEE SA MI ET AL: "Plastid transformation in the monocotyledonous cereal crop, rice (Oryza sativa) and transmission of transgenes to their progeny" MOLECULES AND CELLS, vol. 21, no. 3, June 2006 (2006-06), pages 401-410, XP002531776 ISSN: 1016-8478

### SEQUENCE LISTING

<110> Bayer CropScience SA
<120> TRANSPLASTOMIC PLANTS FREE OF THE SELECTABLE MARKER
<130> BCS 09-4001
<160> 4
<170> PatentIn version 3.4
<210> 1
   <211> 24
   <212> DNA
   <213> Artificial
<220>
   <223> primer
<400> 1
   catgggttct ggcaatgcaa tgtg 24
<210> 2
   <211> 26
   <212> DNA
   <213> artificial
<220>
   <223> primer
<400> 2
   caggatcgaa ctctccatga gattcc 26
<210> 3
   <211> 21
   <212> DNA
   <213> artificial
<220>
   <223> primer
<400> 3
   accacgatcg aacgggaatg g 21
<210> 4
   <211> 20
   <212> DNA
   <213> artificial
<220>
   <223> primer
<400> 4
   cgactacctt ggtgatctcg 20

## Description

The present invention relates to transplastomic plants free of the selectable marker gene, particularly to leguminous plants, to methods for obtaining such plants and to the vectors and constructs used.

Plant transgenesis consists in introducing into a plant one or more genes originating from various organisms (bacteria, viruses, insects, plants), with the aim of providing it with novel characteristics and of improving certain agronomic or food qualities. The great diversity of genes, associated with the development of the conventional genetic transformation techniques, has resulted in the creation of new plant varieties. In certain cases, due to the introduction of characteristics that confer resistance to an herbicide, to pathogens or to various stresses, crop practices can be facilitated and yields increased. In other cases, the nutritive value of the plant and the content of certain essential compounds can be improved.

A large number of crop species belong to the leguminous plant family, in particular protein-yielding plants such as pea, fababean, bean, chickpea, lentils, oil-yielding plants such as soybean and groundnut, and forage such as alfalfa or clover. A fundamental property of leguminous plants, which is greatly responsible for their agronomic value, is their high protein content. This property makes them plants of choice for overexpressing proteins of interest.

Many techniques for obtaining stable transgenic plants consist in introducing the foreign gene into the nuclear genome of the plant. Another means of obtaining transgenic plants is the direct transformation of plastids. Specifically, plastid transformation has many advantages compared to the nuclear transformation, among which mention may be made of:
- Plastid transformation, by which the genes are inserted by double homologous recombination into one or more multiple copies of the circular plastid genome (or plastome) present in each cell, has the advantage of precisely targeting the region of the plastome where it is desired to integrate the gene of interest, avoiding the "positional" effect commonly observed in nuclear transgenesis;
- The obtention of a very large number of copies of the transgene per cell. Specifically, a leaf cell can contain up to 10 000 copies of the plastome. The plant cells can then be manipulated so as to contain up to 20 000 copies of a gene of interest. This feature results in high levels of expression; it being possible for the products of the transgenes to represent more than 40% of the total soluble proteins (De Cosa et al., 2001).
- Plastid transformation has the other advantage of greatly limiting the risk of transgene dispersion into the environment. Since the traits encoded in the plastids are not generally transmissible via pollen, the potential risk of transgene transmission to wild species is limited.

Plastid transformation was initially carried out by biolistic in the unicellular alga *Chlamydomonas reinhardtii* (Boynton et al., 1988), and this approach has been extended to tobacco (Svab et al., 1990 and 1993).
At the current time, stable transformation of the plastids of higher plants is routinely carried out in the tobacco plant *N. tabacum* (Svab and Maliga, 1990; Svab et al., 1993). Some progress has more recently been made with the transformation of plastids from rice (Khan and Maligna, 1999), *Arabidopsis thaliana* (Sikdar et al., 1998), potato (Sidorov et al., 1999), rapeseed (Chaudhuri et al., 1999) and tomato (Ruf et al., 2001). Production of fertile transplastomic soybean plants has been unsuccessful for several years, until the disclosure of a new method and new means for high frequency transformation of soybean plastomes leading to fertile plants (WO 04/053133).
Plastid transformation techniques are more particularly described in the article McBride et al., 1994, in American patents U.S. Pat. Nos. 5,451,513; 5,545,817; 5,545,818 and 5,576,198, and also in international patent applications WO 95/16783 and WO 97/32977.
Plastid transformation has been used to obtain a good level of tolerance to herbicides or resistance to insects, or alternatively for the production of proteins in large amounts. Thus, overexpression, from the tobacco plastome, of genes for tolerance to herbicides such as glyphosate (Daniell, 1998; WO 99/10513; Ye et al., 2000; WO 01/04331, WO 01/04327) or phosphinothricin (Basta) (Lutz et al., 2001) confers excellent tolerance to these herbicides.
Other applications have resulted in the production of transplastomic plants that are tolerant to insects or overproduce therapeutic proteins (McBride et al., 1995; US Patent 5,451,513; Staub et al. (2000); WO 99/10513).

However, one of the main disadvantages of the direct transformation of the plastids of higher plants, such as it is conventionally carried out, is the use of a gene for resistance to an antibiotic as a selectable marker.
The selectable marker generally used for the selection of transplastomic lines is the bacterial gene *aadA,* expressed under the control of plastidial regulatory elements (Svab et al, 1993; Staub et al, 1993). Expression of the *aadA* gene, which encodes an aminoglycoside 3'-adenylyltransferase, confers resistance to two antibiotics, spectinomycin and streptomycin.
The product of the *aadA* gene prevents spectinomycin (or streptomycin) from binding to 16S DNA, a component of the 30S subunit of plastidial ribosomes, involved in recognition of the translation initiation codon, and therefore from inhibiting translation within the plastid. Only the cells that contain plastids expressing the product of the *aadA* gene will be able to continue to grow optimally in vitro and to remain green. An alternative selectable marker is a 16S RNA sequence that has a point mutation that makes it insensitive to spectinomycin.

Unfortunately, this antibiotic also controls bacterial infections in humans and animals. It is therefore preferred not to have it in the final product. Methods that make it possible to eliminate selectable marker genes, in particular antibiotic marker genes, while at the same time keeping the gene of interest present in the transgenic plant, are therefore of major interest.

In this context, and in view of the technical advantages of plastid transformation mentioned above, it is becoming crucial to develop a simple and reliable technique for obtaining transplastomic plants free of the selectable marker, in particular leguminous plants, and more particularly soybean.

A certain number of techniques have been described for eliminating a delectable marker gene, but they are often designed for nuclear transformation and/or are often complex and more or less reliable.
Among these techniques, transposable elements (PCT/US91/04679; Yoder et al 1993) or site-specific recombination systems such as the *cre*/*lox* system of the P1 bacteriophage or the yeast FLP/FRT system (FliPase recombinase; Lyzrik et al., 1997) have been used to remove marker gene integrated by nuclear transformation into the chromosomes.
Site-specific recombination has also been applied to the elimination of a transplastomic marker gene by introduction into the nuclear genome of the plant of a second transgene encoding a CRE protein targeted to the chloroplasts by means of its transit peptide (EP1218488).
In *C*. *reinhardtii* algae, selection methods based on photosynthetic mutants have made it possible to introduce foreign genes of interest into the plastid genome without the use of antibiotic selectable marker genes such as *aadA.* However, these methods cannot be used in higher plants since they are based on the existence of photosynthetic mutants.
A system based on the homologous recombination phenomenon has been used in *Arabidopsis,* in order to remove a marker gene integrated by nuclear transformation (WO 01/96583). In this method, the plants are transformed using a vector which comprises two copies of the gene of interest in the same orientation, surrounding a positive selectable marker gene and a negative selectable marker gene. The positive selectable marker gene makes it possible to select the events that incorporated the transgene into their genome. The two copies of the gene of interest makes it possible to eliminate by homologous recombination the two (positive and negative) selectable marker genes and one of the two copies of the gene of interest. The absence of the negative selectable marker gene in events which have undergone this homologous recombination makes it possible to select them. An example of such a negative selectable marker gene is *CodA* (*Escherichia coli* cytosine deaminase), which deaminates 5-fluorocytosine (non-toxic) to 5-fluorouracil, which is toxic.

In WO 06/07260, the authors have developed a new and reliable strategy to produce marker-free, herbicide-tolerant transplastomic plants. A genetic construct comprising the *aadA* selection cassette inserted between two fragments of the HPPD coding region, corresponding to the NH2-terminus and COOH-terminus of the protein respectively, wherein these two sequences overlap over a fragment P1 of 403 nucleotides, was introduced into the plastome of tobacco plants. Taking advantage of the homologous recombination that may occur inside the plastid genome, the two copies of P1 can recombine inside the plastid genome. This leads to the elimination of the aadA cassette and the restoration of a functional full-length HPPD coding region, conferring DKN resistance to the recombined plastid transgenic plant. Production of plants homoplasmic for the presence of the HPPD gene and absence of *aadA* gene was accelerated and facilitated due to the possible selection on DKN.

The present application discloses new methods and means for the elimination of the selectable marker, making it easier and even more reliable with any gene of interest, even with genes which do not confer a new selectable characteristic to the plants when restored.

### Description of the figures:

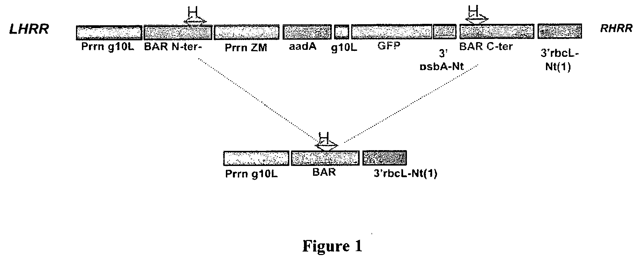

Figure 1 : Construct comprising a dicistronic tandem comprising the *aadA* gene and the *gfp* gene under the control of the *Zea mays* Prrn promoter, inserted between the 5'-terminus sequence (BAR N-term) and the 3'-terminus sequence (BAR C-term) respectively of the gene *bar* under the control of the Tobacco Prrn promoter, and wherein these two sequences overlap over a fragment H having a length sufficient to allow the homologous recombination, the elimination of the dicistronic tandem comprising the *aadA* gene and the *gfp* gene and of one of the two fragments H, and the appearance of a functional *bar* gene (BAR).

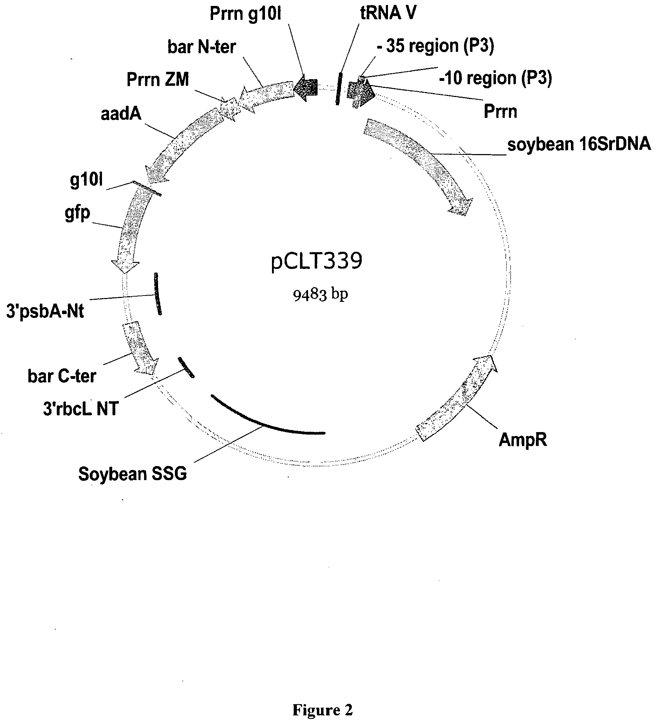

Figure 2: map of the plasmid pCLT339
Figure 3: Molecular analysis of transplastomic soybean lines.
Fig 3a) PCR analysis performed using primers OSSG511 and OSSG311 on a wild-type line and on two transplastomic events regenerated from green-GFP positive sectors (T0 GFP+);
Fig 3b) PCR analysis performed using primers Prrn-Zm-1F and aadA-780R on two transplastomic events regenerated from GFP positive sectors (T0 GFP positive lines) and on six events regenerated from under UV red-GFP negative sectors (T1 GFP negative lines);
Fig 3c) detection assay of the BAR protein performed using a commercial Liberty Link kit on a wild plant and on an event regenerated from under UV red-GFP negative sectors (transplastomic T1 line)

### Detailed description of the invention:

A subject of the present invention is a genetic construction or construct comprising, in the direction of transcription, at least (i) a chimeric gene encoding a selectable marker that confers resistance to a selection agent and (ii) (ii) a reporter gene selected among the list consisting of a chimeric gene encoding a luminescent protein and a chimeric colour gene, both inserted between the 5'-terminus sequence and the 3'-terminus sequence respectively of a chimeric gene of interest, wherein these two sequences overlap over a fragment having a length suitable to allow their recombination.

According to the invention, the "expression ""chimeric gene" or "expression cassette" is intended to mean a nucleotide sequence comprising, functionally linked to one another in the direction of transcription, a regulatory promoter sequence that is functional in plastids, a sequence encoding a protein, and, optionally, a terminator that is functional in the plastids of plant cells.

The term "chimeric gene" or "expression cassette" is generally intended to mean a gene for which certain elements are not present in the native gene, but have been substituted for elements present in the native gene or have been added.
According to the invention, the terms "chimeric gene" or "expression cassette" may also correspond to the case where all the elements of the gene are present in the native gene, and alternatively, the term "gene" may correspond to a chimeric gene. The expression "chimeric gene" or "expression cassette" may also correspond to the case where the sequence encoding a protein is not directly linked to a promoter, but is part, for example, of a polycistronic construct comprising several coding sequences under the control of the same promoter. In that case, each coding sequences under the control of the promoter is designed as a "chimeric gene" or "expression cassette".
According to the invention, the sequence (i) encoding a selectable marker and the (ii) chimeric gene encoding a luminescent protein, or chimeric colour gene, may be part of a polycistronic construct under the control of one promoter.
They may also each be under the control of their own promoter.

According to the invention, the expression "functionally linked to one another" means that said elements of the elemental chimeric gene are linked to one another in such a way that their function is coordinated and allows the expression of the coding sequence. By way of example, a promoter is functionally linked to a coding sequence when it is capable of ensuring the expression of said coding sequence. The construction of the chimeric gene according to the invention and the assembly of its various elements can be carried out using techniques well known to those skilled in the art, in particular those described in Sambrook et al. (2001, Molecular Cloning : A Laboratory Manual (third edition), Nolan C. ed., New York: Cold Spring Harbor Laboratory Press). The choice of the regulatory elements constituting the chimeric gene depends essentially on the plant and on the type of plastid in which they must function, and those skilled in the art are capable of selecting regulatory elements that are functional in a given plant.

Among the promoters that are functional in the plastids of plant cells and that can be used to implement the present invention, mention may be made, by way of example, of the promoter of the *psbA* gene, encoding the D1 protein of PSII (Staub et al., 1993, EMBO Journal 12(2): 601-606) or the constitutive promoter of the ribosomal RNA operon Prrn (Staub et al., 1992) or the tobacco Prrn promoter combined with a 5' portion of the 5' untranslated region of the tobacco *rbcL* gene (Svab et al., 1993). In general, any promoter derived from a plant plastome gene or from a bacterial gene will be suitable, and those skilled in the art are capable of making the appropriate choice from the various promoters available so as to obtain a desired method of expression (constitutive or inducible). A preferred promoter according to the invention comprises the tobacco Prrn promoter combined with a 5'portion of the 5'untranslated region of the tobacco rbcL gene (Svab and Maliga, 1993).

According to the invention, use may also be made, in combination with the promoter, of other regulatory sequences, which may be located between the promoter and the coding sequence, such as transcription activators ("enhancers"), for instance the translation activator of the tobacco mosaic virus (TMV) described in Application WO 87/07644, or of the tobacco etch virus (TEV) described by Carrington & Freed 1990, or the translation activator/ribosome binding site g101 (Ye et al., 2001)

Among the terminators that are functional in the plastids of plant cells, mention may be made, by way of example, of the terminator of the *psbA* gene, of the *rbcL* gene encoding the Rubisco large subunit, or of the *rps16* gene encoding a ribosomal protein of tobacco (Shinozaki et al., 1986; Staub et al., 1993).

A sequence encoding a selectable marker that confers resistance to a selection agent makes it possible to select the plastids and the cells that are effectively transformed, i.e. those that have incorporated the chimeric gene(s) into their plastome. The selection of the transformants is accomplished by culturing the transformed cells or tissues on a medium containing the selection agent.
The selectable marker genes commonly used include the genes encoding genes for resistance to antibiotics, herbicides or to other compounds, which may be lethal for the cells, organelles or tissues that do not express the resistance gene or allele. The selection agent is then the corresponding antibiotic, herbicide or selective compound. If said agent is lethal for the cell, only the transformed cells will live and develop on this medium, whereas the non-transformed cells will die. If the selection agent is not lethal for the cell, the transformed cells and the non-transformed cells will be distinguished by virtue of a different behaviour that may be demonstrated.
A selectable marker may be non-lethal at the cellular level but lethal at the organelle level. For example, the antibiotic spectinomycin inhibits mRNA translation to protein in plastids, but not in the cytoplasm. The tissues containing non-resistant plastids will be whitish whereas the tissues containing resistant plastids will be green. In a dividing cell containing transformed plastids and non-transformed plastids, the non-transformed plastids will disappear under the selection pressure, for the benefit of the transformed plastids, and a population of cells comprising only transformed plastids may be obtained.
The expression "selectable marker gene" is intended to mean a gene encoding a selectable marker, or a chimeric gene encoding a selectable marker.
Among the genes encoding selectable markers, that can be used, mention may be made of genes for resistance to the antibiotics spectinomycin-streptomycin and kanamycin, such as, for example, the chimeric genes *aadA* encoding an aminoglycoside 3"-adenylyltransferase (Svab et al., 1993) and *neo* encoding a neomycin phosphotransferase (Carrer et al., 1993) respectively, but also a gene for tolerance to betaine aldehyde, such as the gene encoding betaine aldehyde dehydrogenase (Daniell et al., 2001), but also genes for tolerance to herbicides, such as the bar gene (White et al., 1990) for tolerance to bialaphos, or the *EPSPS* gene (US 5,188,642) for tolerance to glyphosate.
Preferably, the gene encoding the selectable marker is a gene for resistance to an antibiotic. A preferred gene encoding the selectable marker is the *aadA* gene encoding an aminoglycoside 3"-adenylyltransferase that confers resistance to streptomycin and to spectinomycin (Svab et al., 1993).

In the context of the invention, a chimeric luminescent gene, or luminescent gene, refers to a gene encoding aluminescent protein or peptide.
Therefore, in a particular embodiment, the genetic construction or construct of the invention comprises, in the direction of transcription, at least (i) a chimeric gene encoding a selectable marker that confers resistance to a selection agent and (ii) a chimeric gene encoding a luminescent protein, both inserted between the 5'-terminus sequence and the 3'-terminus sequence respectively of a chimeric gene of interest, wherein these two sequences overlap over a fragment having a length suitable to allow their recombination.

Luminescence refers to a light emitted by sources other than a hot, incandescent body. Luminescence occurs from a body when its atoms are excited by means other than raising its temperature. There are many types of luminescence, including chemiluminescence, produced by certain chemical reactions, chiefly oxidations, at low temperatures; electroluminescence, produced by electric discharges, which may appear when silk or fur is stroked or when adhesive surfaces are separated; and triboluminescence, produced by rubbing or crushing crystals. Bioluminescence is luminescence produced by living organisms and is thought to be a type of chemiluminescence. If the luminescence is caused by absorption of some form of radiant energy, such as ultraviolet radiation or X rays (or by some other form of energy, such as mechanical pressure), and ceases as soon as (or very shortly after) the radiation causing it ceases, then it is known as fluorescence. If the luminescence continues after the radiation causing it has stopped, then it is known as phosphorescence.
According to the invention, the term luminescence is considered as a general term, which encompasses the different types of luminescence, as fluorescence, bioluminescence, chemiluminescence, phosphorescence....
In the context of the invention, a sequence encoding a luminescent protein may make it possible to identify the plastids and the cells that have been transformed and which have conserved the expression cassette comprising the sequence encoding the selectable marker and the sequence encoding the luminescent protein. More interesting, disappearance of the luminescence (i.e. non-luminescence) for a cell previously selected as having been transformed makes it possible to identify the plastids and the cells that have eliminated through homologous recombination the expression cassette comprising the sequence encoding the selectable marker and the sequence encoding the luminescent protein.

According to the invention, the terms "luminescent protein" refer to a protein which is able to produce a light when its atoms are excited by means other than raising its temperature.

Several luminescent proteins are known and the genes encoding them known and already used as reporter genes.

Preferably, the luminescent protein is the green fluorescent protein (GFP) (Sidorov et al., 1999). The green fluorescent protein is a protein, originally isolated from the jellyfish *Aequorea victoria,* that fluoresces green when exposed to blue light (Tsien R, 1998). The GFP from *A. victoria* has a major excitation peak at a wavelength of 395 nm and a minor one at 475 nm. Its emission peak is at 509 nm which is in the lower green portion of the visible spectrum. The GFP from the sea pansy *Renilla reniformis* has a single major excitation peak at 498 nm. In cell and molecular biology, the GFP gene is frequently used as a reporter of transformation and/or expression (Phillips G (2001).

Other examples of luminescent protein include the enzyme luciferase. Luciferases are enzymes commonly used in nature for biolurninescence. The most famous one is firefly luciferase (EC 1.13.12.7) from *Photinus pyralis,* although the enzyme exists in organisms as different as the Jack-O-Lantern mushroom *(Omphalotus olearius)* and many marine creatures. In the luciferase reaction, light is emitted at 562nm when luciferase acts on the appropriate luciferin substrate. The reaction is very energy efficient: nearly all of the energy input into the reaction is transformed into light. Photon emission can be detected by light sensitive apparatus such as a luminometer, modified optical microscopes, or a sensitive CCD (charge-coupled device) camera.

The invention also relates to a method of obtaining transplastomic plant cells or plants free of selectable marker, in particular antibiotic selectable marker, comprising at least the following steps:
a) transforming at least one plant cell with a vector suitable for the transformation of plastids comprising at least (i) a chimeric gene encoding a selectable marker that confers resistance to a selection agent and (ii) a chimeric gene encoding a luminescent protein, both inserted between the 5'-terminus sequence and the 3'-terminus sequence respectively of a chimeric gene of interest, wherein these two sequences overlap over a fragment having a length suitable to allow their recombination;
b) culturing the cell(s) comprising the transformed plastids on a first medium comprising the selection agent;
c) culturing the cell(s) on a second medium that does not comprise the selection agent;
d) selecting the non-luminescent cells or plants.

According to the invention, the expression "vector suitable for the transformation of plastids" may refer, by way of example, to a transformation vector comprising two regions for homologous recombination of the plastome of the plant, bordering a genetic construction or construct according to the invention.
These regions, located upstream (LHRR) and downstream (RHRR) of the elemental chimeric gene(s), allow double homologous recombination with an intergenic region of the plastome which comprises the contiguous LHRR and RHRR regions.
According to the invention, the sequences homologous with a zone of the plastome to be transformed correspond to sequences exhibiting 80% identity with the corresponding sequences in the plastome to be transformed, preferably 90% identity, preferably 95%, and preferably 99% identity. According to a preferred embodiment of the invention, the sequences homologous with a zone of the plastome to be transformed correspond to sequences exhibiting 100% identity with the corresponding sequences in the plastome to be transformed.

Preferably, the two homologous recombination regions according to the invention correspond to contiguous sequences that allow the integration of the chimeric gene into an intergenic region of the plastome. In a particular embodiment, this region corresponds to the region of the plastome ribosomal RNA operon. In another particular embodiment, this intergenic region comprises the 3' end of the rbcL gene encoding the Rubisco large subunit, and the other homologous sequence comprises the 5' end of the *accD* gene, encoding a subunit of acetyl-CoA-carboxylase, and more particularly, this intergenic region comprises the 3' end of the *rbcL* gene encoding the Rubisco large subunit corresponding to nucleotides 57755 to 59297 of the plastome of *N. tabacum,* cv. Petit Havana, and the other homologous sequence comprises the 5' end of the *accD* gene corresponding to nucleotides 59298 to 60526 of the plastome of *N*. *tabacum,* cv. Petit Havana.
The choice of these two homologous recombination regions may depend upon the plant to be transformed. The preferred homologous regions according to the plant to be transformed are well known from the skilled man. As an example, preferred region for the transformation of the plastome of leguminous plant are the region of the ribosomal RNA operon of the plastome.

In another particular embodiment, the invention refers to a chimeric colour gene. A chimeric colour gene, or colour gene, refers to a gene which is able to confer a specifc colour to the plant cell, to the plant or to part of the plant. Such colour genes can be genes expressing a pigment, or genes regulating the expression of such pigment. Examples of pigment are the polyphenols compounds, phlobaphene or proanthocyanidin, which are synthesized through the flavonoid biosynthesis pathway (Himi E. et at., 2005, Eupliytica, Vol 143, N°3, pp. 239-242). Many of these colour genes are known from the skilled man. As examples, one can cited *Ph6* gene or related genes that influence the anthocyanin pigmentation in Petunia plant (Chuck G. et al., 1993), the R and *Rc* genes which are controlling the pigmentation of grain and coleoptile (E. Himi, 2005, Genome, vol 48, N° 4, pp. 747-754 (8); E. Himi et al., 2005, J Exp Bot 53: 1569-1574), the *My*bgene which is known as regulating the anthocyanin biosynthesis in plant (Takos A. M. et al., 2006).

The invention also relates to a method of obtaining transplastomic plant cells or plants free of selectable marker, in particular antibiotic selectable marker, comprising at least the following steps:
a) transforming at least one plant cell with a vector suitable for the transformation of plastids comprising at least (i) a chimeric gene encoding a selectable marker that confers resistance to a selection agent and (ii) a colour gene, both inserted between the 5'-terminus sequence and the 3'-terminus sequence respectively of a chimeric gene of interest, wherein these two sequences overlap over a fragment having a length suitable to allow their recombination;
b) culturing the cell(s) comprising the transformed plastids on a first medium comprising said selection agent;
c) culturing the cell(s) on a second medium that does not comprise said selection agent;
d) selecting the non-coloured plant cells or plants.

In the meaning of the invention, the non-coloured (or wild-coloured) cells or plants of the step d) are plant cells or plants, which are not harboring the colour specifically due to the expression of the colour gene of step a).

In the meaning of the invention, the (i) chimeric gene encoding a selectable marker that confers resistance to a selection agent and, the (ii) chimeric gene encoding a luminescent protein, or chimeric colour gene, are both inserted between the 5'-terminus sequence and the 3'-terminus sequence respectively of a chimeric gene of interest, wherein these two sequences overlap over a fragment H having a length suitable to allow their recombination. Fragment H therefore constitutes a direct repeat sequence surrounding the chimeric genes (i) and (ii) and having a length suitable for recombination. When recombination between these two direct repeat sequences occurs, the chimeric genes (i) and (ii) and one of these repeat sequences H are eliminated, leading to the restoration of a full-length,chimeric gene of interest.

In a particular embodiment of the invention, the gene of interest is not present as a full-length entity in the construct of the invention, i.e. before the recombination occurs. Accordingly, in a particular embodiment of the constructs, methods, transplastomic plant cells, plants or seeds of the invention, the gene of interest is not present as a full-length entity.

In another particular embodiment of the invention, the full-length gene of interest is present in the construct of the invention, i.e. before the recombination occurs. In this particular embodiment, the construct of the invention comprises, in the direction of transcription, at least (i) a chimeric gene encoding a selectable marker that confers resistance to a selection agent and (ii) a chimeric colour gene or a chimeric gene encoding a luminescent protein, both inserted between two direct repeats of a chimeric gene of interest.

In these two particular embodiments of the invention, the removal of the selection marker is transparent for the cell, meaning than no sequence used for that purpose remains in the cell after recombination. An example of such construct of the invention is given in Figure 1.

Homologous recombination is a mechanism that occurs naturally and at a relative high frequency inside the plastid genome. Such mechanism is possible when a repeat sequence of a suitable size is present in the genome. In a preferred embodiment, the repeat sequence has a length superior or equal to 90 nucleotides. A repeat sequence of less than 90 nucleotides may be used, but the frequency of recombination then decreases considerably (Eibl C, 1999).
A direct repeat sequence is a sequence of nucleic acids that is duplicated and the duplicated sequence of which is oriented in the same direction as the original sequence, and not in the opposite direction.

Preferably, the repeat sequence is a sequence of at least 50 nucleotides, more preferably of at least 90 nucleotides.

The construction according to the invention can be carried out using techniques well known to those skilled in the art, in particular those described in Sambrook et al. (1989, Molecular Cloning: A Laboratory Manual, Nolan C. ed., New York: Cold Spring Harbor Laboratory Press). It may also be completely or partially synthetic and produced by conventional chemical synthesis techniques.

According to the invention, the term "transplastomic plant cells or plants" is intended to mean plant cells or plants that have stably integrated into their plastome a chimeric gene that is functional in plastids, in particular in chloroplasts. The plastome consists of the genome of the cellular organelles other than the nucleus, in particular the chloroplasts genome.

To implement the method according to the invention, the transformation step can be carried out on embryogenic tissues obtained from immature embryos of plants.
Preferably, the embryogenic tissues are calli or any other tissue containing cells which have conserved a totipotent state.

The transformation of the cells can be carried out by any method of transforming plant cells. Among the transformation methods that can be used to obtain transformed cells according to the invention, one of these consists in bringing the cells or tissues of the plants to be transformed into contact with polyethylene glycol (PEG) and with the transformation vector (Chang and Cohen, 1979, Mol. Gen. Genet. 168(1), 111-115; Mercenier and Chassy, 1988. Biochimie 70(4), 503-517). Electroporation is another method which consists in subjecting the cells or tissues to be transformed and the vectors to an electric field (Andreason and Evans, 1988, Biotechniques 6(7), 650-660; Shigekawa and Dower, 1989, Aust, J. Biotechnol. 3(1), 56-62). Another method consists in directly injecting the vectors into the cells or the tissues by microinjection (Gordon and Ruddle, 1985). The transformation of plant cells or tissues may also be carried out by means of bacteria of the *Agrobacterium* species, preferably by infection of the cells or tissues of said plants with *A. tumefaciens* (Knopf, 1979, Subcell. Biochem. 6, 143-173; Shaw et al., 1983, Gene 23(3):315-330, lshida et al. (1996, Nat. Biotechnol. 14(6), 745-750) or *A. rhizogenes* (Bevan and Chilton, 1982; Tepfer and Casse-Delbart, 1987) that have been genetically modified, thus allowing the targeting of the T-DNA specifically to the plastids. According to a preferred embodiment of the method according to the invention, the method referred to as particle bombardment or biolistic method will be used. It consists in bombarding the tissues with particles onto which the vectors according to the invention are adsorbed (Bruce et al., 1989; Klein et al., 1992; US Patent No. 4,945,050).

After transformation, a selection step carried out using a first culture medium comprising the selection agent corresponding to the selectable marker gene used makes it possible to select the transformation events that have integrated the exogenous DNA into the plastid genome. For example, if the *aadA* gene is used as selectable marker gene, the selection medium used will comprise spectinomycin and/or streptomycin. The material capable of growing on this medium will be propagated and/or regenerated while maintaining this spectinomycin and/or streptomycin selection so as to obtain tissues or plants that contain the exogenous DNA in all the plastid genomes
In a subsequent step, the cells or tissues selected on the first culture medium are placed in a second medium, referred to as non-selective medium, so as to make it possible to eliminate the gene encoding the selectable marker and to obtain a complete and functional gene of interest by recombination between the repeat sequences.
The term "non-selective medium" is intended to mean a medium that does not contain the selection agent which is present on the first culture medium.

Then, in a particular embodiment of the invention, the luminescence of the cells is observed using an appropriate mean well known by the skilled man and depending on the nature of the luminescence, and the non-luminescent cells or tissues are selected. These non-luminescent cells are those for which a recombination has occurred between the two direct repeat sequences, leading to the elimination of the chimeric gene (i) encoding a selectable marker that confers resistance to a selection agent, of the chimeric gene (ii) encoding the luminescent protein and of one of these two repeat sequences, and to the restoration of a functional full-length chimeric gene of interest.

In another particular embodiment of the invention, the plant cells which are not harbouring the colour specifically due to the expression of the colour gene are selected.
Nevertheless, the colour specifically due to the expression of the colour gene may be visible only or also lateron, for the plant or for part of the plant which has been regenerated from plant cells obtained on the medium that does not comprise the first-medium selection agent, and selection of plants free of selectable marker may occur at that level, when the non-coloured features as defined above is detectable.

Additionally to this last step, the elimination of the selectable marker may be confirmed by testing the sensitivity of the cells to the selection agent, and/or by testing the expression of the gene of interest, and/or by using molecular biology techniques such as Southern blotting-type hybridization and the PCR technique. All these technologies, which are more complicated than the observance of non-luminescence, non-colour or growth in a specific medium, are well known by the skilled man.

The culture media used are well known to those skilled in the art. They are in particular described in Gamborg *et al.* (1968) and Murashige *et al.* (1962)*.*

Once selected, the cells may be used in order to regenerate the Transplastomic plants which express the gene of interest and which are free of the selectable marker. Alternatively, selection may occur after regeneration based on the non-luminescent, or non-colour of the plants.

A "functional full-length chimeric gene of interest" denotes a gene of interest capable of being expressed and of encoding a peptide or a functional protein. In addition, according to the invention, it is a gene that has been reconstituted following the excision of the genes encoding (i) a selectable marker and (ii) a chimeric gene encoding a luminescent protein, or chimeric colour gene, and of one of the two repeat sequences by homologous recombination.

The gene of interest may be any gene introduced into the plant cell or plant so as to confer on it a specific advantage.

In a particular embodiment of the invention, the chimeric gene of interest is a gene conferring resistance to an herbicide.

According to a more particular embodiment of the invention, the chimeric gene of interest that confers a specific advantage is the *bar* gene (C.J. Thompson, et al.) which confers tolerance to bialaphos , or a synthetic *bar* gene encoding the PAT enzyme, the amino acid sequence of which differs from the amino acid sequence encoded by the natural gene (White et al., 1990; EP257542).

According to another more particular embodiment of the invention, the "chimeric gene of interest encodes a hydroxyphenylpyruvate dioxygenase (HPPD).
Hydroxyphenylpyruvate dioxygenases (HPPDs) are enzymes that catalyse the reaction of conversion of para-hydroxyphenylpyruvate (HPP) to homogentisate (Crouch N.P. & al., Tetrahedron, 53, 20, 993-7010, 1997). Certain molecules that inhibit the hydroxyphenylpyruvate dioxygenase (HPPD) have found a use as herbicides (Pallett K.E. et al. 1997, Pestic. Sci. 50 83-84). Such herbicides having HPPD as a target, that are described in the state of the art, are especially isoxazoles (EP 418 175, EP 470 856, EP 487 352, EP 527 036, EP 560 482, EP 682 659, US 5 424 276), in particular isoxaflutole (IFT), a herbicide selective for maize, diketonitriles or DKNs (EP 496 630, EP 496 631), in particular 2-cyano-3-cyclopropyl-1-(2-SO₂CH₃-4-CF₃phenyl)propane-1,3-dione and 2-cyano-3-cyclopropyl-1-(2-SO₂CH₃-4-2,3-Cl₂₋phenyl)propane-1,3-dione, triketones (EP 625 505, EP 625 508, US 5,506,195), in particular sulcotrione or mesotrione, or else pyrazolinates.

The term "HPPD" is intended to mean any native, mutated or chimeric HPPD enzyme exhibiting the HPPD activity. Many HPPDs are described in the literature, in particular the HPPDs of bacteria such as *Pseudomonas* (Rüetschi & al., Eur. J. Biochem., 205, 459-466, 1992, WO 96/38567), of plants, for instance *Arabidopsis* (WO 96/38567, Genebank AF047834) or of carrot (WO 96/38567, Genebank 87257),of *Coccicoides* (Genebank COITRP) or of mammals such as humans, mice or pigs.
Advantageously, a mutated HPPD is an HPPD mutated so as to obtain properties of tolerance to HPPD-inhibiting herbicides, such as the mutated HPPDs described in patent application WO 99/24585.
The term "chimeric HPPD" is intended to mean an HPPD comprising elements from various HPPDs, in particular the chimeric HPPDs described in patent application WO 99/24586.

In another more particular embodiment of the invention, the chimeric gene of interest encodes a 5-enolpyruvylshikimate-3-phosphate synthase (EPSPS).
EPSPS is a plastid enzyme involved in the shikimate biosynthetic pathway, resulting in the synthesis of aromatic amino acids. EPSPS is known to be the target enzyme of herbicides of the family of phosphoriic acids of phosphonomethylglycine type.
Sequences encoding EPSPSs which are naturally tolerant, or used as such, with respect to herbicides of the phosphonomethylglycine family, in particular with respect to glyphosate, are known. By way of example of genes encoding tolerant EPSPS enzymes, mention may be made of the sequence of the *AroA* gene of the bacterium *Salmonella typhimurium* (Comai et al., 1983, Science 221, 370-371), the sequence of the *CP4* gene of the bacterium Agrobacterium sp. (WO 92/04449), or the sequences of the genes encoding the EPSPS of Petunia (Shah et al., 1986, Science 233,478-481), of tomato (Gasser et al., 1988, J. Biol. Chem. 263, 4280-4289) or of Eleusine (WO 01/66704).
Sequences encoding EPSPSs that have been made-tolerant to glyphosate by mutation are also known. By way of example, mention may be made of the sequences of the genes encoding mutated EPSPSs of bacterial origin (Stalker et al., 1985, J. Biol, Chem. 260(8), 4724-4728) or of plant origin (EP 0293358; Ruff et al., 1991, Plant Physiol. 96(S), Abstract 592; WO 91/04323; WO 92/06201; EP 0837944).

In another particular embodiment of the invention, the chimeric gene of interest encodes an insecticidal toxin, for example a gene encoding a *Bacillus thuringiensis* (Bt) insecticidal protein. Such genes encoding a Bt insecticidal protein are well known from the skilled man, and may be easily find with their sequences in N. Crickmore et al., 1998, as well as through the websites: http://www.lifesci.susx.ac.uk/home/Neil Crickmore/Bt/, and http://www.lifesci.susx.ac.uk/home/Neil Crickmore/Bt/vip.html,

The chimeric gene of interest may also be a gene for resistance to diseases, for example a gene encoding the oxalate oxydase enzyme as described in patent application EP 0 531 498 or US patent 5,866,778, or a gene encoding another antibacterial and/or antifungal peptide, such as those described in patent applications WO 97/30082, WO 99/24594, WO 99/02717, WO 99/53053 and WO 99/91089. It is also possible to introduce genes encoding plant agronomic characteristics, in particular a gene encoding a delta-6 desaturase enzyme as described in US patents 5,552, 306 and 5,614, 313 and patent applications WO 98/46763 and WO 98/46764, or a gene encoding a serine acetyltransferase (SAT) enzyme as described in patent applications WO 00/01833 and WO 00/36127.

The chimeric gene of interest may also encode a protein of pharmaceutical or veterinary interest. By way of example, such a protein may be an anticoagulant (serum protease, hirudin), an interferon or human serum albumin. The proteins produced by the plants according to the invention may also be antibodies, or proteins used as a basis for vaccines.

In a preferred embodiment of the invention, the methods related to the invention and constructs of the invention are applied to dicotyledonous plants.

In a particular embodiment of the invention, the methods related to the invention and constructs of the invention are applied to leguminous plants.

According to the present invention, the term "leguminous plant" is intended to mean a plant of the Fabaceae family: Preferred leguminous plants according to the invention are the leguminous plants of agronomic interest, such as pea (Pisum sativum), broadbean (Vicia faba major), faba bean (Vicia faba minor), lentils (Lens culinaris), bean (Phaseolus vulgaris), chickpea (Cicer arietinum), soybean (Glycine max), groundnut (Arachis hypogea), alfalfa (Medicago sativa) or clover (Trifolium sp.).

According to a preferred embodiment of the invention, the methods related to the invention and constructs of the invention are applied to soybean, Glycine max.

The invention also relates to a transformation vector suitable for the transformation of plant plastids, characterized in that it comprises a construct according to the invention.

The plastid transformation and regeneration of the leguminous cells can be carried out by any method of transforming and regenerating leguminous plant cells. Suitable methods are described in WO 04/053133.

Among the sequences encoding a protein of interest which can be integrated into the transplastomic plant cells or plants according to the invention, mention may be made of the coding sequences of genes encoding an enzyme for resistance to a herbicide, such as, for example, the *bar* gene (C.J. Thompson, et al., 1987) which confers tolerance to bialaphos , a synthetic *bar* gene encoding the PAT enzyme (White et al., 1990; EP257542), a gene encoding an EPSPS enzyme (WO 97/04103) which confers tolerance to glyphosate, or a gene encoding an HPPD enzyme (WO 96/38567) which confers tolerance to isoxazoles. Mention may also be made of a gene encoding an insecticidal toxin, for example *Bacillus thuringiensis* (Bt) insecticidal protein (N. Crickmore et al., 1998; http://www.lifesci.susx.ac.uk/home/Neil Crickmore/Bt/; http://www.lifesci.susx.ac.uk/home/Neil Crickmore/Bt/vip.html;

It is also possible to introduce into these plants genes for resistance to diseases, for example a gene encoding the oxalate oxydase enzyme as described in patent application EP 0 531 498 or US patent 5,866, 778, or a gene encoding another antibacterial and/or antifungal peptide, such as those described in patent applications WO 97/30082, WO 99/24594, WO 99/02717, WO 99/53053 and WO 99/91089. It is also possible to introduce genes encoding plant agronomic characteristics, in particular a gene encoding a delta-6 desaturase enzyme as described in US patents 5,552, 306 and 5,614, 313 and patent applications WO 98/46763 and WO 98/46764, or a gene encoding a serine acetyltransferase (SAT) enzyme as described in patent applications WO 00/01833 and WO 00/36127.
According to a particular embodiment of the invention, the transplastomic leguminous plants according to the invention may be transformed with an expression cassette encoding a protein of pharmaceutical or veterinary interest. By way of example, such a protein may be an anticoagulant (serum protease, hirudin), an interferon or human serum albumin. The proteins produced by the plants according to the invention may also be antibodies, or proteins used as a basis for vaccines.

A subject of the invention is also a transplastomic plant cell, plant or a part of this plant, and the progeny of this plant, containing a construct according to the invention.
Preferably, a transplastomic leguminous plant cell, plant or a part of this plant, and the progeny of this plant, containing a construct according to the invention; More preferably, a transplastomic leguminous plant cell of agronomic interest, plant or a part of this plant, and the progeny of this plant such as pea (Pisum sativum), broadbean (Vicia faba major), faba bean (Vicia faba minor), lentils (Lens culinaris), bean (Phaseolus vulgaris), chickpea (Cicer arietinum), soybean (Glycine max), groundnut (Arachis hypogea), alfalfa (Medicago sativa) or clover (Trifolium sp.); Even more preferably, a transplastomic soybean (Glycine max) plant cell, plant or a part of this plant, and the progeny of this plant,.

A subject of the invention is also a transplastomic plant cell, plant or a part of this plant, and the progeny of this plant, containing a transgene of interest and free of selectable marker, that can be obtained by means of one of the methods described above.
Preferably, a transplastomic leguminous plant cell, plant or a part of this plant, and the progeny of this plant, containing a transgene of interest and free of selectable marker. This transplastomic leguminous, plant cell, plant or a part of this plant, and the progeny of this plant, may be obtained by means of one of the methods described above; More preferably, a transplastomic leguminous plant of agronomic interest, plant or a part of this plant, and the progeny of this plant, such as pea (Pisum sativum), broadbean (Vicia faba major), faba bean (Vicia faba minor), lentils (Lens culinaris), bean (Phaseolus vulgaris), chickpea (Cicer arietinum), soybean (Glycine max), groundnut (Arachis hypogea), alfalfa (Medicago sativa) or clover (Trifolium sp.); Even more preferably, a transplastomic soybean (Glycine max) plant cell, plant or a part of this plant, and the progeny of this plant.

The term "part of this plant" is intended to mean any organ of this plant, whether it is aerial or subterranean. The aerial organs are the stems, the leaves and the flowers comprising the male and female reproductive organs. The subterranean organs are mainly the roots, but they may also be tubers. The term "progeny" or "descendants" is intended to mean mainly the seeds containing the embryos derived from the reproduction of these plants with one another. By extension, the term "progeny" or "descendants" applies to all the seeds formed at each new generation derived from crosses in which at least one of the parents is a transformed plant according to the invention. Descendants may also be obtained by vegetative multiplication of said transformed plants. The seeds according to the invention may be coated with an agrochemical composition comprising at least one active product having an activity selected from fungicidal, herbicidal, insecticidal, nematicidal, bactericidal or virucidal activities.

The present invention also relates to any products such as the meal or the oil, which are obtained by processing the plants, part thereof, or seeds of the invention. For example, the invention may encompasses oil obtained from the processing of leguminous plant of the invention, or grains obtained from the processing of the seeds according to the invention, but also meal and food obtained from the further processing of the seeds, the grains, or the plants, as well as any food product obtained from said meal.

The invention also refers to the use of a construct according to the invention for obtaining a transplastomic plant cell or plant free of selectable marker; Preferably, for obtaining a transplastomic leguminous plant cell or plant; More preferably, for obtaining a Transplastomic leguminous plant of agronomic interest, such as pea (Pisum sativum), broadbean (Vicia faba major), faba bean (Vicia faba minor), lentils (Lens culinaris), bean (Phaseolus vulgaris), chickpea (Cicer arietinum), soybean (Glycine max), groundnut (Arachis hypogea), alfalfa (Medicago sativa) or clover (Trifolium sp.); Even more preferably, for obtaining a transplastomic soybean (Glycine max) plant cell or plant.

The molecular biology techniques that may be useful to implement the invention are described in Ausubel (Ed.), Current Protocols in Molecular Biology, John Wiley and Sons Inc. (1994): Maniatis T., Fritsch E.F. and Sambrook J. Molecular Cloning: A Laboratory Manual, Cold Spring Harbor laboratory, Cold Spring Harbor, NY (1989). The PCR reactions are carried out in a Perkin Elmer GeneAmp PCR system 9600 device. The amplification reactions for each sample are carried out in the course of 30 cycles comprising various steps: a denaturation step at 94°C for one minute, a pairing step of 45 seconds at a temperature of 50 to 60°C, depending on the primers used, and an elongation step at 72°C for 1 to 2 minutes depending on the size of the PCR products to be amplified. These cycles are preceded by a denaturation period at 94°C lasting 5 minutes, and followed by a final elongation period of 5 minutes at 72°C. A temperature of 4°C is applied after 30 cycles. The PCR products are separated on an agarose gel.

The invention will be more particularly illustrated by the examples that follow, it being understood that these examples are not limiting.

### Examples

### Example 1: Construction of plastid transformation vectors for the elimination of the marker gene

### a) Construction of pCLT339 (comprising aadA + gfp)

Soybean plastid transformation vector pCLT339 was derived from pCLT323 (Dufourmantel et al., 2007 - Plant Biotechnol. J. 5(1): 118-133.).
A synthetic sequence was synthezised (pCLT435) containing convenient restriction sites for cloning into pCLT323 (XhoI and SwaI). The sequence contains a dicistronic expression cassette linking the aadA selection marker and gfp under the control of the corn 16S rDNA plastid promoter. This cassette disrupts a non-functional plastid bar expression cassette which encodes as upstream component (453 bp) the N-terminal fragment of BAR (Nter) and as downstream element (458 bp) the C-terminal fragment of BAR (C-ter). These two uncomplete BAR fragments have a direct overlap of 367 nucleotides. The bar and gfp sequences were codon-optimized in order to fit the tobacco Plastid codon usage.

### b) Construction of pCLT340 (comprising aadA, without gfp)

A derived soybean plastid transformation vector lacking the gfp reporter gene was constructed. This vector pCLT340 was obtained directly from pCLT339 cut with SalI and SpeI, blunt-ended and religated.

### Example 2: Production of transplastomic soybean plants

Transplastomic soybean events (cv. Jack) were generated for vectors pCLT339 and pCLT340 using spectinomycin as selection agent according to the method described in patent WO/2004/053133 and in Dufourmantel et al. (2004) Plant Mol. Biol. 55(4): 479-489).
The selected events were examined under UV illumination and displayed uniform expression of the reporter gene in all examined organs for pCLT339 and obviously not for pCLT340. Separation of leaf proteins from 1 regenerated plant (pCLT339) followed by Coomassie-blue staining and western blot clearly shows that this line strongly expresses GFP.

### Example 3: Elimination of the selectable marker gene and evaluation of the restoration of the bar gene

Transplastomic plants (T0 generation) were transferred to the greenhouse and set seeds normally. Embryogenic cultures from immature embryos were initiated on the progenies according to Dufourmantel et al. (2004). Some proliferating calli were then exposed to various concentrations of L-phosphinotricin (basta) in order to favour the growth of plant cells having eliminated the aadA-gfp antibiotic-marker by homologous recombination between bar-Nter and bar-Cter (which would then restore a functional herbicide bar tolerance gene). For material corresponding to pCLT339, the generated calli were examined under UV. Whereas a strong majority of the embryogenic cultures displayed uniform GFP expression, a few % of the examined calli, whether or not exposed to basta, clearly revealed red sectors indicating, a loss of GFP.
The red sectors were separated and propagated further in vitro, then regenerated into plants (T1 generation) which were transferred to the greenhouse.

### Example 4: Molecular analysis of the lines

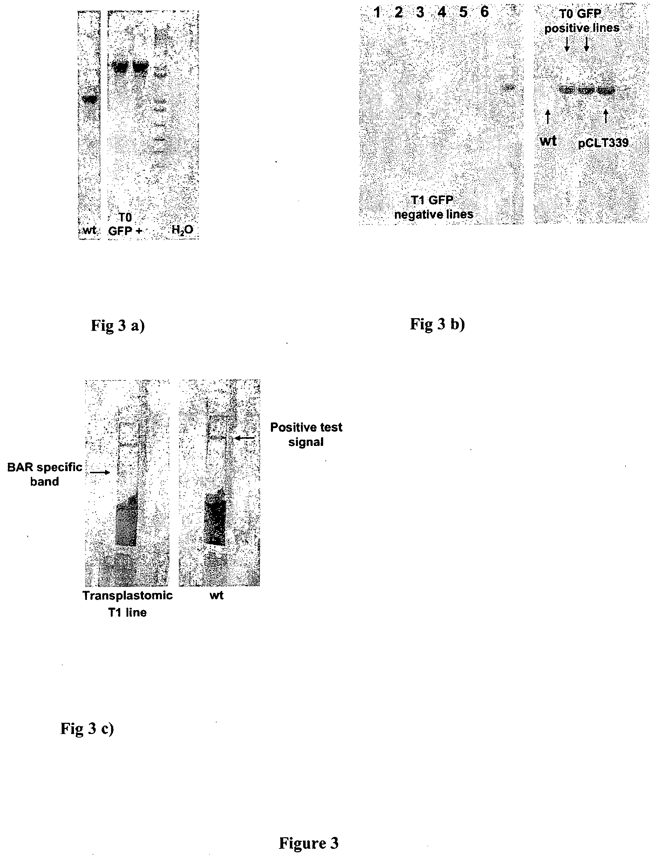

Selected transgenic line with pCLT339 were analyzed at the DNA level using PCR performed with different couples of primers.
The first PCR analysis was performed on two T0 events selected for pCLT339 with primer pair OSSG511 (5'-CATGGGTTCTGGCAATGCAATGTG-3') and OSSG311 (5'-CAGGATCGAACTCTCCATGAGATTCC-3'). The primers land on both sides of the plastid genome insertion site and allow therefore a precise determination of the level of homoplasmy of the selected material. This analysis clearly shows that there is no signal corresponding to wild-type plastid genome in the two analyzed transplastomic lines, indicating that the selected lines are homoplasmic for the transgenes (Figure 3a)
A second amplification was performed on two T0 events (GFP positives) and six T1 events which were regenerated from under UV red GFP-negative sectors. The primer pair Prrn-Zm-1F (5'-ACCACGATCGAACGGGAATGG-3') and aadA-780R (5'-CGACTACCTTGGTGATCTCG-3') allows the amplification of the aadA selection marker cassette. The result of this analysis shows that there is no presence anymore of the aadA antibiotic selection marker in the GFP-negative regenerated soybean lines (Figure 3b)

A detection assay of the BAR protein was performed using a commercial LL (Liberty Link) kit. The result shows that a specific signal is observed in extracts made from transplastomic soybean, showing that the elimination of the aadA-gfp cassette has precisely occurred between the bar direct repeats restauring a functional herbicide tolerance expression cassette (figure 3c).

### Example 5: Field trial

Field trials were performed with the transplastomic line in comparison with the commercial LL 27 and LL55 lines, wherein the *bar* gene is introduced in the nuclear genome of the plant and encodes a cytoplasmic phosphinothricin acetyltransferase (PAT).

Plants were sprayed with the commercial herbicide Ignite® over the top of above varieties at the agronomic level of 450 g a.i./ha when soybeans were at V4 stage of growth.
Phyto assessments was conducted 14 DAA (Days After Application).
The transplastomic line displayed better tolerance to glufosinate than any other soybean variety in this test
LL27 and LL55 lines exhibited typical yellowing of leaves which was commercially acceptable. The transplastomic did not have this discoloration.

### BIBLIOGRAPHY

*Andreason and Evans, Biotechniques 6(7) (1988), 650-660
* Austin E.A. et al., 1993, Journal of bacteriology, vol. 175, no11, pp. 3685-3686
*Bevan and Chilton, Annu. Rev. Genet. 16: (1982), 357-384
*Boynton J.E., Gillham N.W., Harris E.H., Hosler J.P., Jones A.R., Randolph-Anderson B.L., Robertson D., Klein T.M., and Shark K.B. Science 240 (1988), 1534-1538
*Bruce et al, Proc. Natl. Acad. Sci. USA 86(24), (1989) 9692-9696
*Carrer H. et al. Mol. Gen. Genet. 241: (1993), 49-56
*Chang and Cohen, Mol. Gen. Genet. 168(1) (1979), 111-115
*Chaudhuri S. (1999). WO 00/39313
* Chuck G. et al.(1993) The plant cell, vol 5, N° 4, pp. 371-378
*Comai et al, Science 221: (1983), 370-371
* Crickmore N. et al., 1998, Microbiology and molecular biology reviews, p807-813
*Crouch et al, Tetrahedron, (1997), 53, 20,6993-7010
*Daniell H., Datta R., Varma S., Gray S., and Lee S.B. Nat. Biotechnol. 16 (4): (1998) 345-348
*Daniell H. et al. Curr Genet. 39: (2001) 109-116
*De Cosa B., Moar W., Lee S.B., Miller M., and Daniell H. Nat. Biotechnol. 19 (1): (2001). 71-74
* Eibl C. et al., 1999, Plant J 19: 333-345
*Fischer N., Stampacchia O., Redding K., and Rochaix J.D. Mol. Gen. Genet. 251: (1996). 373-380
*Gasser et al, J. Biol. Chem., 263: (1988), 4280-4289
*Gamborg et al, Exptl Cell Res 50, (1968), 151-158
*Gordon and Ruddle, Gene 33(2), (1985), 121-136
*Himi E. et al., Euphytica, 2005, Vol 143, N°3, pp. 239-242
*Himi E. et al., 2005, Genome, vol 48, N° 4, pp. 747-754 (8);
*Himi E. et al., 2005, J Exp Bot 53: 1569-1574
*Ishida et al, Nat. Biotechnol. 14(6), (1996), 745-750
*Khan M.S. and Maliga P. Nat. Biotechnol. 17, (1999). 910-915
*Klein et al, Biotechnology 10(3), (1992), 286-291
*Knopf, Subcell. Biochem. 6, (1979), 143-173
*Lutz K.A., Knapp J.E., and Maliga P. Plant Physiol. 125(4): (2001), 1585-1590
*McBride K.E., Svab Z., SchaafD.J., Hogan P.S., Stalker D.M., and Maliga P. Biotechnology 13(4): (1995). 362-365
*Murashige et al, Physiologia Plancarum 15, (1962), 473-497
*Mercenier and Chassy, Biochimie 70(4), (1988), 503-517
* Naested et al., 1999 plant J; 18(5), pp. 571-576
*Padgette S.R. et al, J. Biol. Chem., 266: (1991), 33
*Pallett et al, pestc. Sci. 50: (1997), 83-84
*Phillips G (2001) FEMS Microbiol Lett 204 (1): 9-18
*Rüetschi et al, Eur. J. Biochem. 205, (1992), 459-466
*Ruf et al, Plant Physio/. 96(S) (1991), Abstract 592
*Ruf S., Hermann M., Berger I.J., Carrer H., and Bock R. Nat. Biotechnol. 19(9): (2001). 870-875
* Sambrook et al. (2001) Molecular Cloning : A Laboratory Manual (third edition), Nolan C. ed., New York: Cold Spring Harbor Laboratory Press
*Shah et al, Science 233: (1986), 478-481
*Shaw et al, Gene 23(3): (1983), 315-330
*Shigekawa and Dower, Aust. J. Biotechnol. 3(I), (1989), 56-62
*Shinozaki K., Ohme M., Tanaka M., Wakasugi T., Hayashida N., Matsubayashi T., Zaita N., Chunwongse J., Obokata J., Yamaguchi-Shinozaki K., Ohto C., Torasawa K., MengB.Y., Sugita M., Deno H., Kamogashira T., Yamada K., Kusuda J., Takaiwa F., Kato A., Tdhdoh N., Shimada H., and Suguiara M. EMBO J. 5: (1986). 2043-2049
*Sidorov V.A., Kasten D., Pang S.Z., Hajdukiewicz P.T., Staub J.M., and Nehra N.S. Plant J. 19(2): (1999). 209-216
*Sikdar S.R., et al. Plant Cell Reports 18: (1998). 20-24
* Stalker et al, J. Biol. Chem. 260(8), (1985), 4724-4728
*Staub et al, Plant Cell 4: (1992), 39-45
*Staub et al, EMBO Journal 12(2): (1993),601-606
*Staub J.M., Garcia B., Graves J., Hajdukiewicz P.T., Hunter P., Nehra N., Paradkar V., Schlitter M., Carroll J.A., Spatola L., Ward D., Ye G., and Russell D.A. Nat. Biotechnol. 18(3): (2000). 333-338
*Svab Z., Hajdukiewicz P., and Maliga P. Proc. Natl. Acad. Sci. USA 87(21): (1990). 8526-8530
*Svab et al, Proc. Natl. Acad. Sci. 90: (1993), 913-917
* Takos A. M. et al., 2006, Plant Physiol. 142:1216-1232
*Tepfer and Casse-Delbart, Microbiol. Sci. 4(1), (1987), 24-28
* Thompson C.J. et al., EMBO Journal, vol.6 no.9 pp.2519-2523, 1987
* Tsien R (1998). Annu Rev Biochem 67: 509-44
*White et al, Nucleic Acid Res. 18(4): (1990), 1062
*Ye G.N., Hajdukiewicz P.T., Broyles D., Rodriguez D., Xu C.W., Nehra N., and Staub J.M. Plant J. 25(3): (2001). 261-270

## Claims

1. A construct comprising, in the direction of transcription, at least (i) a chimeric gene encoding a selectable marker that confers resistance to a selection agent and (ii) a reporter gene selected among the list consisting of a chimeric gene encoding a luminescent protein and a chimeric colour gene, both inserted between the 5'-terminus sequence and the 3'-terminus sequence respectively of a chimeric gene of interest, wherein these two sequences overlap over a fragment having a length suitable to allow their recombination.

2. The construct according to claim 1 wherein the gene of interest is not present as a full length entity.

3. The construct according to claim 1 or 2 wherein the luminescent protein is a green fluorescent protein or a luciferase.

4. The construct according to claims 1 to 3 wherein the chimeric gene of interest is the *bar* gene, a gene encoding a hydroxyphenylpyruvate dioxygenase (HPPD), a gene encoding a 5-enolpyruvylshikimate-3-phosphate synthase (EPSPS), or a gene encoding a *Bacillus thuringiensis (Bt)* insecticidal protein.

5. A transplastomic plant cell containing a construct according to any one of the claims 1 to 4.

6. A plant cell according to claim 5 which is a dicotyledonous transplastomic plant cell.

7. A plant cell according to claim 6 which is a leguminous transplastomic plant cell, more preferably a soybean plant cell.

8. A transplastomic plant **characterized in that** it contains a transplastomic plant cell according to claim 7.

9. A transplastomic plant according to claim 8 which is a soybean plant.

## Patentansprüche

1. Konstrukt, das in Transkriptionsnchtung mindestens Folgendes umfasst: (i) ein chimäres Gen, das für einen Selektionsmarker codiert, der Resistenz gegenüber einem Selektionsmittel verleiht, und (ii) ein Reportergen, ausgewählt aus der Liste bestehend aus einem chimären Gen, das für ein lumineszierendes Protein codiert, und einem chimären Farbgen, die beide zwischen der 5' -terminalen Sequenz bzw. der 3'-terminalen Sequenz eines chimären Gens von Interesse insertiert sind, wobei diese beiden Sequenzen über ein Fragment mit einer Länge, die geeignet ist, ihre Rekombination zu gestatten, überlappen.

2. Konstrukt nach Anspruch 1, wobei das Gen von Interesse nicht als Volllängen-Einheit vorhanden ist.

3. Konstrukt nach Anspruch 1 oder ?, wobei es sich bei dem lumineszierenden Protein um ein grün fluoreszierendes Protein oder eine Luciterase handelt.

4. Konstrukt nach den Ansprüchen 1 bis 3, wobei es sich bei dem chimären Gen von Interesse um das bar-Gen, ein Gen, das für eine Hydroxyphenylpyruvatdioxygenase (HPPD) codiert, ein Gen, das für eine 5 '-Enolpyruvylshikimat-3-phosphatsynthase (EPSPS) codiert, order ein Gen, das für ein insektizides Protein aus *Bacillus thuringiensis* (Bt) codiert, handelt.

5. Transplastomische Pflanzenzelle, die ein Konstrukt nach einem der Ansprüche 1 bis 4 enthält.

6. Pflanzenzelle nach Anspruch 5, bei der es sich um eine transplastomische dikotyle Pflanzenzelle handelt.

7. Pflanzenzelle nach Anspruch 6, bei der es sich um eine transplastomische Leguminosenpflanzenzelle, stärker bevorzugt eine Sojabohnenpflanzenzelle, handelt.

8. Transplastomische Pflanze, **dadurch gekennzeichnet, dass** sie eine transplastomische Pflanzenzelle nach Anspruch 7 enthält.

9. Transplastomische Pflanze nach Anspruch 8, bei der es sich um eine Sojabohnenpflanze handelt.

## Revendications

1. Construction comprenait, dans la direction de la transcription, au moins (i) un gène chiwère codant pour un marqueur sélectionnable, qui confère une résistance à un agent de selection, et (ii) un gène rapporteur, choisi dans la liste constituée par un gène chimère codant pour une protéine luminescente et un gène de couleur chimère, tous deux insérés entre la séquence terminale en 5' et la séquence terminale en 3' respectivement d'un gène chimère d'intérêt, dans lequel ces deux séquences se chevauchent sur un fragment ayant une longueur appropriés pour permettre leur recombinaison.

2. Construction selon la revendication 1, dans laquelle le gène d'intérêt n'est pas présent en tant qu'entité de longueur totale.

3. Construction selon la revendication 1 ou la 2, dans laquelle la protéine luminescente est une protéine fluorescente verte ou une luciférase.

4. Construction selon les revendications 1 à 3, dans laquelle le gène chimère d'intérêt est le gène *bar*, un gène codant pour une hydroxyphénylpyruvate dioxygénase (HPPD), un gène codant pour une 5-énolpyruvylshikimate-3-phosphate synthase (EPSPS) ou un gène codant pour une protéine insecticide de *Bacillus thuringiensis* (*Bt*).

5. Cellule de plante transplastomique contenant, une construction selon l'une quelconque des revendications 1 à 4.

6. Cellule végétale, selon la revendication 5, qui est une cellule de plane dicotylédone transplastomique.

7. Cellule végétale, selon la revendication 6, qui est une cellule de plante légumineuse transplastomique, plus préférentiellement une cellule de plant de soja.

8. Plante transplastomique **caractérisée en ce qu'**elle continent une cellule végétale transplastomique selon la revendication 7.

9. Plante transplastomique, selon la revendication 8, qui est une plante de soja.
